# EUROPÄISCHE PATENTSCHRIFT

(11) **EP 2 179 710 B1**
(45) Veröffentlichungstag und Bekanntmachung des Hinweises auf die Patenterteilung: **07.09.2011**
(21) Anmeldenummer: 09013317.4
(22) Anmeldetag: 21.10.2009
(51) Int. Cl.: A61F 5/00, A61F 5/01

(54) **Orthese zur Behandlung pathologischer Deviationen im Rückfuss**
Orthosis for handling pathologic deviations in hind-foot
Orthèse de traitement de déviations pathologiques à l'arrière du pied

(30) Priorität: 21.10.2008 DE 102008052369; 21.10.2008 DE 102008052517
(43) Veröffentlichungstag der Anmeldung: 28.04.2010
(73) Patentinhaber: Pohlig GmbH, 83278 Traunstein (DE)
(72) Erfinder: Pohlig, Kurt, 83278 Traunstein (DE)
(74) Vertreter: Paustian, Othmar

(56) Entgegenhaltungen:
- EP-A- 1 867 307
- WO-A-91/13604
- SU-A1- 1 454 461

## Beschreibung

Die Erfindung betrifft eine Orthese zur Behandlung pathologischer Deviationen im Rückfuß.

Der Rückfuß umfasst insbesondere das Sprungbein (Talus) und das Fersenbein (Calcaneus). Projiziert in die Transversalebene liegen physiologische talocalcaneale Winkel im Bereich von etwa 30° bis etwa 35°.

Bei bestimmten Deviationen im Rückfuß liegen unter anderem abweichende talocalcaneale Winkel vor. So ist dieser Winkel bei dem so genannten Klumpfuß kleiner und bei dem Knickfuß größer.

Es ist bekannt, entsprechende Deviationen im Rückfuß operativ oder konservativ zu behandeln.

Zur konservativen Behandlung des Klumpfußes ist der so genannte Klumpfußgips bekannt. Hier wird i.d.R. ein Oberschenkelgips angelegt, um eine hinreichende Redression des Fußes nach außen zu erzielen. Damit der Fuß mit dem Klumpfußgips als Ganzes gegenüber dem Oberschenkel nach außen redressiert werden kann, muss das Kniegelenk gebeugt sein. Es ist auch bekannt, eine entsprechend geformte und starre Orthese anzulegen.

Weiter ist bei einem solchen Verfahren eine Redression von 70° bis 80° abweichend von der Neutral-Null-Stellung (s.u.) erforderlich, da sich die Drehung über verschiedene Teile des Beins verteilt; dies belastet insbesondere den Bandapparat im gesamten Bein.

Bei der Klumpfußbehandlung nach Ponseti erfolgt eine Korrektur schrittweise über das sukzessive Tragen von mehreren, bspw. drei bis acht, Gipsen für jeweils unterschiedliche Redressionen.

Der Erfindung liegt die Aufgabe zu Grunde, eine Orthese anzugeben, mit der Deviationen im Rückfuß besonders gezielt und einfach behandelt werden können.

Die Aufgabe wird gelöst durch eine Orthese zur Behandlung pathologischer Deviationen im Rückfuß, mit einer oberen Fixiereinrichtung zum Fixieren der Sprungbeinrolle in der Knöchelgabel und einem Fußteil, das zur Aufnahme der subtalaren Fußplatte eingerichtet ist und relativ zu der oberen Fixiereinrichtung in der Transversalebene zwischen mindestens zwei Ausrichtung drehbar und in jeder der Ausrichtungen lösbar fixierbar ist.

Bevorzugte Ausgestaltungen der Erfindung sind in abhängigen Ansprüchen angegeben und werden im Folgenden näher erläutert.

Die Erfindung beruht auf der Erkenntnis, dass zur konservativen Korrektur der Winkelstellung zwischen dem Sprungbein und der darunter liegenden subtalaren Fußplatte, zu welcher das Fersenbein gehört, es vor allem der Fixierung der Sprungbeinrolle, als Teil des Sprungbeins, in ihrer Einbettung an der Knöchelgabel bedarf. Dazu dient die oberen Fixiereinrichtung.

Weiter beruht die Erfindung auf der Idee, die subtalare Fußplatte relativ zu dem fixierten Sprungbein in der Transversalebene drehbar und bei hinreichender Redression fixierbar in der Orthese aufzunehmen. Dazu weist die Orthese ein Fußteil auf, welches zur Aufnahme zumindest der subtalaren Fußplatte, oder auch des ganzen Fußes, eingerichtet ist und welches relativ zu der Fixiereinrichtung der Orthese in der Transversalebene drehbar und in verschiedenen Ausrichtungen, zumindest in einer Fehlstellung und in einer Korrekturstellung, in der Transversalebene lösbar fixierbar ist.

Wird auf eine Drehung des Fußteils in der Transversalebene abgestellt, so heißt dies nicht, dass die Erfindung auf eine Bewegung in der Transversalebene beschränkt ist und keine weiteren Komponenten aufweisen darf. Vielmehr wird unter einer Drehung in der Transversalebene jede Bewegung verstanden, bei der die Projektion in die Transversalebene im Ergebnis an einer Drehung teilnimmt. Sinngemäß gilt das gleiche auch für weiter unten erwähnte Drehungen des Fußteils in weiteren Ebenen. So kann bei einer bevorzugten Ausführungsform bspw. mit einer Drehung des Vorfußes nach oben auch eine Spitzfußkomponente behandelt werden.

Die Ausrichtung des Fußteils kann relativ zu der so genannten Neutral-Null-Stellung angegeben werden. Diese Stellung nimmt der Fuß im Normalfall beim aufrechten Stand des Menschen ein.

Zur Korrektur bspw. eines Klumpfußes wird der Fuß in Fehlstellung in dem Fußteil aufgenommen und die Sprungbeinrolle in der Knöchelgabel über die Fixiereinrichtung fixiert werden. Anschließend kann das Fußteil in der Transversalebene nach außen gedreht und dort fixiert werden.

Zur Korrektur eines Knickfußes wird das Fußteil nach innen gedreht. Angestrebt wird letztlich ein physiologischer Winkel zwischen dem Fersenbein und dem Sprungbein. Zur Behandlung bietet sich jedoch, zumindest zunächst, eine moderate Überkorrektur an. Bei hinreichender Redression wird dabei das Fußteil fixiert; diese Ausrichtung des Fußteils wird solange beibehalten, wie es die Behandlung erfordert.

Vorzugsweise ist das Fußteil jedoch zwischen mehr als zwei Ausrichtungen in der Transversalebene drehbar und in jeder dieser Ausrichtungen lösbar fixierbar, idealerweise ist die Ausrichtung des Fußteils in der Transversalebene sogar kontinuierlich einstellbar und dabei in jeder seiner Ausrichtungen lösbar fixierbar. Die Drehung des Fußteils kann etwa durch manuelle Führung an dem distalen Ende des Fußes erfolgen.

Insgesamt ermöglicht es die Erfindung, pathologische Deviationen im Rückfuß besonders gezielt und einfach zu behandeln. Strukturen, die nicht in unmittelbarer Nähe der zu korrigierenden Strukturen liegen, werden geschont. Zumindest bei günstigem Behandlungsverlauf kann auf einen Gips verzichtet werden.

Weiter ist es möglich, die Redression des Fußes im Laufe der Behandlung an den Fortschritt derselben anzupassen. Insbesondere erübrigt sich hier ggf. das Anlegen mehrerer Gipse nacheinander.

Außerdem ermöglicht die Orthese ein Anlegen in der Fehlstellung, was die Handhabbarkeit steigert.

Bei einer bevorzugten Ausführungsform der Erfindung ist das Fußteil in der Transversalebene zweidimensional, also insb. nach vom und hinten und nach links und nach rechts, verschiebbar und in jeder dieser Positionen lösbar fixierbar. Auf diese Weise ist es möglich, bei beliebiger Drehung des Fußteils, die Orthese so einzustellen, dass der biomechanische Drehpunkt im Sinus Tarsi - in der Mitte zwischen der Furche zwischen dem Sprung- und Fersenbein - liegt, seiner physiologischen Lage entsprechend.

Auch eine Anordnung des Gelenks zur Drehung des Fußteils in der Transversalebene unterhalb eines Abschnitts zur Aufnahme der Ferse des Fußteils unterstützt dabei eine Einstellung der Orthese so, dass der biomechanische Drehpunkt im Sinus Tarsi verbleiben kann.

Ggf. kann auch schon eine Orthese vorteilhaft eingesetzt werden, bei der das Fußteil lediglich medio-lateral entlang einer Geraden quer zu der medialen Ebene verschiebbar und entlang dieser Geraden lösbar fixierbar ist.

Vergleichbar zu der Sprachregelung bei den Drehungen, wird unter einer Verschiebung in der Transversalebene jede Bewegung verstanden, deren Projektion in die Transversalebene an einer Verschiebung in derselben teilnimmt.

Um die Orthese noch flexibler einsetzen zu können, ist es bevorzugt, sie so auszulegen, dass das Fußteil auch in der Sagittal- und/oder Frontalebene drehbar und lösbar fixierbar ist, also Drehbarkeit in jede Raumrichtung, etwa den anatomischen Achsen folgend, gegeben ist. Die entsprechenden Drehungen können auch aus anderen Bewegungen zusammengesetzt sein, etwa aus einer Kipp- und einer Translationsbewegung. So kann der Fuß in die therapeutisch erforderliche Position gedreht werden, ohne beispielsweise das Sprungbein in eine Fehlposition zu drängen. Idealerweise ist das Fußteil auch bezüglich dieser Drehungen für ein Kontinuum von Ausrichtungen lösbar fixierbar. Es sind so beliebige Pronationen und Supinationen einstellbar.

Es sollten jedoch, vor allem in der Sagittalebene, keine zu großen Abweichungen von der Neutral-Null-Stellung eingestellt werden, da ansonsten das Risiko für eine Luxation zu hoch ist. So sollte etwa der Winkel zwischen dem Fuß und dem Unterschenkel, bei gleichzeitiger Drehung in der Transversalebene, 90° nicht wesentlich überschreiten, da ansonsten die Sprungbeinrolle aus der Knöchelgabel springen kann. Anders ausgedrückt, vorzugsweise beträgt der Winkel zwischen Fuß und Unterschenkel im redressierten Zustand höchstens 120°; besser höchstens 100°.

Vorzugsweise weist die oberen Fixiereinrichtung ein Schalenteil, oder auch mehrere Schalenteile, für den Unterschenkel auf, welches den Innen- und den Außenknöchel zur mechanischen Sicherung der Sprungbeinrolle in physiologischer Position distal umklammert. Der Talus kann so über die Knöchelgabel gesichert werden. Über die Umklammerung wird genügend Druck für eine hinreichende Fixierung ausgeübt. Dazu kann das Schalenteil etwa mit Riemen, ggf. mit Klettverschluss, zusammengeschnürt werden. Zur Schonung der Knöchel, können diese etwa mit einem ringförmigen Polymergelkissen geschützt werden und/oder aber auch mit einer tubusförmigen Innenhülle. Auch ansonsten können das oder die Schalenteile zur Verbesserung des Komforts mit Innenhüllen versehen sein. Innenhüllen werden in der Orthopädietechnik auch "Liner" genannt.

Die Schalenteile können etwa aus kohlefaserverstärktem Gießharz bzw. einem Gießharzlaminat hergestellt werden. Für die Innenhüllen hat sich Polyethylenschaum bewährt.

Vorzugsweise weist das Fußteil ein Schalenteil zum Halten des Fußes, bzw. zumindest der subtalaren Fußplatte, auf. Einen sicheren Halt gewährt etwa ein Fußteil, welches den Fuß, bspw. im Bereich der Mittelfußknochen, ringförmig umschließt. Dieses Schalenteil kann auch mit einer Innenhülle ausgestattet sein. Für einen leichten Einstieg in die Orthese kann das Fußteil fersenseitig mit einer abnehmbaren Klappe versehen sein.

Bei einer bevorzugten Ausführungsform ist das Fußteil an einer Halterung befestigt, die ihrerseits an der oberen Fixiereinrichtung befestigt ist. Dabei ist die Halterung als U-förmiger Auftrittsbügel ausgebildet, dessen beide Schenkel an der oberen Fixiereinrichtung befestigt sind und dessen geschlossenes Ende sich unter dem Fußteil befindet, wobei das Fußteil an dem Auftrittsbügel mittels eines Gelenks drehbar gestützt ist.

Die Halterung kann etwa durch zwei seitlich verlaufende an der oberen Fixiereinrichtung starr befestigte Schienen verwirklicht sein. Im unteren Bereich können die Schienen miteinander verbunden sein und den Auftrittsbügel bspw. einstückig ausbilden. Der Auftrittsbügel ist vorzugsweise fix relativ zu der oberen Fixiereinrichtung.

Das Fußteil kann auch an anderen Teilen der Orthese mittels eines Gelenks drehbar gestützt sein. Auch ein Auftrittsbügel muss nicht unbedingt gegeben sein.

Vorzugsweise handelt es sich bei dem Gelenk um ein Kugelgelenk.

Ist ein Auftrittsbügel gegeben, ist es bevorzugt, das Gelenk in der Transversalebene verschiebbar sowie lösbar fixierbar an dem Auftrittsbügel zu befestigen.

Vor allem für Behandlungsverfahren und Operationstechniken im Zusammenhang mit flexiblen knöchernen Strukturen, etwa erkrankten, abgenutzten oder operierten Gelenken, kann es wünschenswert sein, diese knöchernen Strukturen gezielt entlastet halten oder sogar auseinanderziehen zu können.

Bekannt ist zum Beispiel der "Fixateur externe" (frz.; "äußerer Festhalter") zum Ruhigstellen eines Knochenbruches oder zur Knochenverlängerung. Hier werden Pins auf beiden Seiten des Knochenbruchs beziehungsweise der auseinanderzuziehenden knöchernen Struktur durch die Haut und das sonstige Gewebe in den Knochen getrieben und dort verankert. Üblicherweise werden die Pins über Stangen außerhalb des Körpers fest miteinander verbunden. Soll ein Knochen verlängert werden, so wird sukzessive eine Callusbildung immer wieder angeregt. Dabei werden die Pins über die Stangen sukzessive zunehmend auseinander gedrückt. Die Anwendung eines Fixateur externe ist recht belastend.

Bevorzugt ist eine Orthese nach der Erfindung auch zum gestreckten Halten, insbesondere Auseinanderziehen, flexibler knöcherner Strukturen eines Körpers, insbesondere eines intraartikulären Spalts, mit einer ersten Befestigungseinrichtung zum Fixieren der Orthese an dem Körper auf einer Seite der knöchernen Struktur, einer zweiten Befestigungseinrichtung zum Fixieren der Orthese an dem Körper auf der, bezogen auf die Streckerichtung, anderen Seite der knöchernen Struktur, und einer die erste Befestigungseinrichtung und die zweite Befestigungseinrichtung miteinander verbindenden Einstelleinrichtung, welche dazu ausgelegt ist, durch ihre Betätigung wahlweise einen verkürzten oder einen verlängerten Abstand zwischen der ersten und der zweiten Befestigungseinrichtung herzustellen.

Die erste Befestigungseinrichtung kann etwa der oberen Fixiereinrichtung entsprechen und die zweite Befestigungseinrichtung dem Fußteil; oder andersherum.

Beliebige flexible knöcherne Strukturen - also nicht knöchern miteinander verwachsen Strukturen - können mit einer Orthese gut fixiert werden; auch gegen den Widerstand von Bändern und Muskeln. Weiter beruht die bevorzugte Ausführungsform auf der Idee, eine Orthese für einen gestreckten Halt auf den zwei gegenüberliegenden Seiten einer flexiblen knöchernen Struktur mit je einer Befestigungseinrichtung, also mehrteilig, auszulegen sowie die Befestigungseinrichtungen über eine Einstelleinrichtung zum Herstellen unterschiedlicher Abstände zwischen den Befestigungseinrichtungen miteinander zu verbinden.

Vor allem verlangt die Orthese keinen Eingriff in das Gewebe. Sie kann einfach äußerlich angelegt werden. In der Regel wird es besonders einfach sein, die Orthese bei verkürztem Abstand zwischen den Befestigungseinrichtungen anzulegen und nach dem Anlegen über die Einstelleinrichtung, soweit gewünscht, den verlängerten Abstand herzustellen.

Die flexible knöcherne Struktur kann mit einer entsprechenden Orthese im gestreckten Zustand ruhig gehalten werden. Durch einen hinreichend verlängerten Abstand zwischen den Befestigungseinrichtungen kann die flexible knöcherne Struktur zwischen den Befestigungseinrichtungen auch auseinander gezogen werden; nicht knöchern miteinander verwachsene Strukturen werden aufgedehnt.

Das Auseinanderziehen flexibler knöcherner Strukturen kann diese vorteilhaft entlasten bzw. die korrekten anatomischen Verhältnisse können ggf. so hergestellt werden.

Grundsätzlich kann die Orthese vorteilhaft am menschlichen oder auch am tierischen Körper eingesetzt werden.

Flexible knöcherne Strukturen können etwa beliebige Gelenke mit intraartikulärern Spalt oder Pseudarthrosen, korrespondierende von einem Zwischenraum abgehende Gelenkteile oder Frakturen sein. Grundsätzlich kann jede nicht knöchern verwachsene Struktur durch die Orthese gedehnt bzw. auseinandergezogen werden.

Beispielsweise kann eine entsprechende Orthese bei folgenden Indikationen entweder zur Reposition, Distraktion oder Mobilisation vorteilhaft eingesetzt werden:

Betreffend das obere Sprunggelenk, und das untere Sprunggelenk etwa bei Osteachondrosis dissecans (konservativ und postoperativ), postoperativ nach Knorpeltransplantation, zur Interimsbehandlung bei Endoprothesenwechsel (septisch) und bei Endprothesen mit postoperativen Mobilisierungsproblemen.

Grundsätzlich kann man im Fall der Algodystrophie (nach M. Sudeck) zur schonenden Anwendung einer Mobilisierung die Orthese einsetzen.

Allgemein kann eine entsprechende Orthese bei Arthrose, postoperativer Nachbehandlung, Ausheilung oder auch ansonsten bei Entzündungen und/oder Schmerzen sinnvoll einsetzbar sein bzw. auch zur gezielten Knochenverlängerung oder Achsenkorrektur.

Besonders vorteilhaft ist die Möglichkeit, bei der Behandlung einer pathologischen Deviation im Rückfuß flexible knöcherne Strukturen gestreckt halten beziehungsweise auseinander ziehen zu können. Beispielsweise kann bei einem postoperativen Klumpfuß der Abstand zwischen Fußteil und oberen Fixiereinrichtung an eine gegebenenfalls vorhandene Schwellung angepasst werden. Ist etwa eine Arthrose in Verbindung mit einer Deviationen im Rückfuß gegeben, kann neben der Behandlung der Korrektur eine dauerhafte Dehnung in Streckrichtung sinnvoll sein.

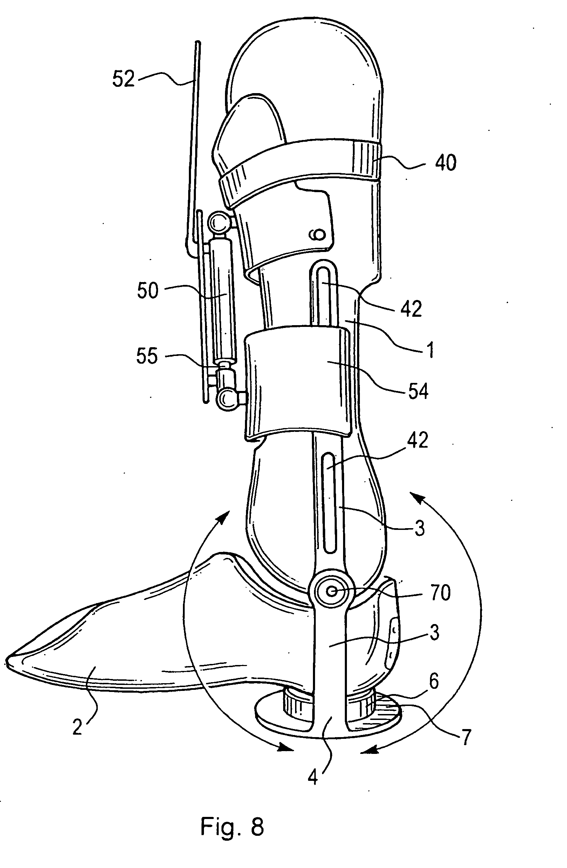

Bei dieser bevorzugten Ausführungsform ist es auch grundsätzlich möglich, neben einem Auseinanderziehen der Befestigungseinrichtungen, deren Position auch quer zur Streckrichtung relativ zueinander einzustellen (vgl. Fig. 8).

Vorzugsweise kann über die Einstelleinrichtung nicht nur ein verkürzter und ein verlängerter Abstand, sondern mehrere weitere Abstände, idealerweise sogar ein Kontinuum von Abständen, zwischen den Befestigungseinrichtungen eingestellt werden.

Vorzugsweise ist die Orthese dazu ausgelegt, frei wählbare Abstände zwischen der ersten und der zweiten Befestigungseinrichtung herzustellen, soweit diese in ein Intervall von einem minimalen Abstand bis zu einem maximalen Abstand fallen.

Diese Auslegung ermöglicht es etwa, den Abstand zwischen den Befestigungseinrichtungen dem Fortschritt einer Behandlung entsprechend nachzuführen.

Bei einer bevorzugten Ausführungsform ist eine Schiene in Streckrichtung unbeweglich, idealerweise starr, mit der ersten Befestigungseinrichtung verbunden. Die zweite Befestigungseinrichtung kann in dieser Schiene geführt werden. Es können etwa zwei als Schienen fungierende metallische Streben gegenüberliegend seitlich an der ersten Befestigungseinrichtung befestigt und in Richtung zweite Befestigungseinrichtung ausgerichtet sein. Zur Führung der zweiten Befestigungseinrichtung in den Schienen können diese jeweils eine länglichen Spalt aufweisen, in welche die zweite Befestigungseinrichtung etwa über Führungsstifte eingreift.

Eine Führung der Relativbewegung der Befestigungseinrichtungen in Streckrichtung lässt sich über eine Schiene konstruktiv einfach und stabil realisieren.

Vorzugsweise umfasst die Einstelleinrichtung eine Schaltfedereinrichtung, also eine federbelastete Einrichtung, die zwischen verschiedenen Zuständen hin- und hergeschaltet werden kann. In einem Schaltzustand wird über die Schaltfedereinrichtung der verkürzte Abstand zwischen den Befestigungseinrichtungen eingestellt; in einem anderen Schaltzustand der verlängerte Abstand zwischen diesen.

Bei der Schaltfedereinrichtung kann es sich um eine Art von Totpunktfeder handeln. Die Schaltfedereinrichtung kann etwa einen Arbeitszylinder, insbesondere eine Gasdruckfeder, umfassen.

Eine Verlängerung bzw. eine Verkürzung der Einstelleinrichtung kann bei einem Arbeitszylinder durch Ausfahren bzw. Einziehen der Kolbenstange umgesetzt werden. Wird etwa die erste Befestigungseinrichtung an dem dem Zylindergehäuse abgewandten Ende der Kolbenstange befestigt und die zweite Befestigungseinrichtung an dem Zylindergehäuse, so schlagen sich die Zustandänderungen des Arbeitszylinders in Abstandänderungen zwischen den Befestigungseinrichtungen nieder.

Es ist auch bevorzugt, eine Einstelleinrichtung mit einer Gewindestange einzusetzen. Hier kann beispielsweise die erste Befestigungseinrichtung derart mit der Gewindestange verbunden sein, dass keine Relativbewegung zwischen Gewindestange und der ersten Befestigungseinrichtung in Streckrichtung möglich ist. Die andere Befestigungseinrichtung kann dann zur Einstellung des Abstands, etwa über eine drehbar gehaltene Mutter, an dem Gewinde entlanggeführt werden. Grundsätzlich können auch Zahnstangen oder Schnecken für die Einstelleinrichtung verwendet werden.

Vorzugsweise ist die Einstelleinrichtung dazu ausgelegt, den Abstand zwischen der ersten Befestigungseinrichtung und der zweiten Befestigungseinrichtung starr herzustellen. Einmal eingestellt, soll ein bestimmter Abstand zwischen den Befestigungseinrichtungen bis zur nächsten Einstellungsänderung unveränderlich sein.

Alternativ ist es bevorzugt, die Einstellungseinrichtung so auszulegen, dass der Abstand zwischen der ersten Befestigungseinrichtung und der zweiten Befestigungseinrichtung federnd-nachgiebig hergestellt ist. Hier ist der Abstand zwischen den Befestigungseinrichtungen auch bei einer bestimmten Einstellung der Einstelleinrichtung nicht fix. Wird die Orthese in Streckrichtung zusammengedrückt, so wird der Abstand zwischen den Befestigungseinrichtungen gestaucht. Lässt die Belastung nach, so werden die Befestigungseinrichtungen über eine Federeinrichtung wieder auseinander gedrückt. Als Federeinrichtung kann die Einstelleinrichtung etwa eine Feder oder einen Gasdruckzylinder aufweisen.

Wird der Abstand federnd nachgiebig hergestellt, kann auch die Kraft, mit der die Befestigungseinrichtungen auseinander gezogen werden, vorgegeben werden.

Weiter ist es bevorzugt, für die Befestigungseinrichtungen anatomisch formschlüssige Schalenteile zu verwenden, welche den Körper auf jeweils einer Seite der knöchernen Struktur zumindest abschnittsweise umfassen, idealerweise vollständig.

Die Schalenteile können etwa aus kohlefaserverstärktem Gießharz bzw. einem Gießharzlaminat hergestellt werden. Für die Innenhüllen hat sich Polyethylenschaum bewährt.

Vorzugsweise ist die Unterschenkelschale so ausgelegt, dass sie an dem Schienbeinplateau und den Schienbeinkondylen abgestützt werden kann. Weiter weist sie ein Fußteil zum Fixieren der Orthese an dem Fuß mit einer zumindest abschnittsweisen ringförmigen Fassung für diesen auf.

Die in der vorangehenden und der folgenden Beschreibung offenbarten Einzelmerkmale können auch in anderen als den gezeigten Kombinationen erfindungswesentlich sein.

Im Folgenden soll die Erfindung auch anhand von Ausführungsbeispielen näher erläutert werden, ohne dabei die Erfindung durch die Beispiele einschränken zu wollen:
Figur 1 zeigt nebeneinander Teile einer ersten erfindungsgemäßen Orthese.
Figur 2 zeigt eine Vorderansicht der ersten erfindungsgemäßen Orthese in zusammengebautem Zustand.
Figur 3 zeigt eine Rückansicht der Orthese aus Figur 2.
Figur 4 zeigt eine Seitenansicht der Orthese aus den Figuren 2 und 3.
Figur 5 zeigt in einer Rückansicht den unteren Abschnitt einer zweiten erfindungsgemäßen Orthese.
Figur 6 zeigt eine Orthese zum Auseinanderziehen knöcherner Strukturen.
Figur 7 zeigt noch eine Orthese zum Auseinanderziehen knöcherner Strukturen.
Figur 8 zeigt eine Seitenansicht einer Orthese zur Behandlung pathologischer Deviationen im Rückfuß und zum Auseinanderziehen knöcherner Strukturen.

Für gleiche bzw. einander entsprechende Merkmale werden figurenübergreifend die gleichen Bezugszeichen verwendet.

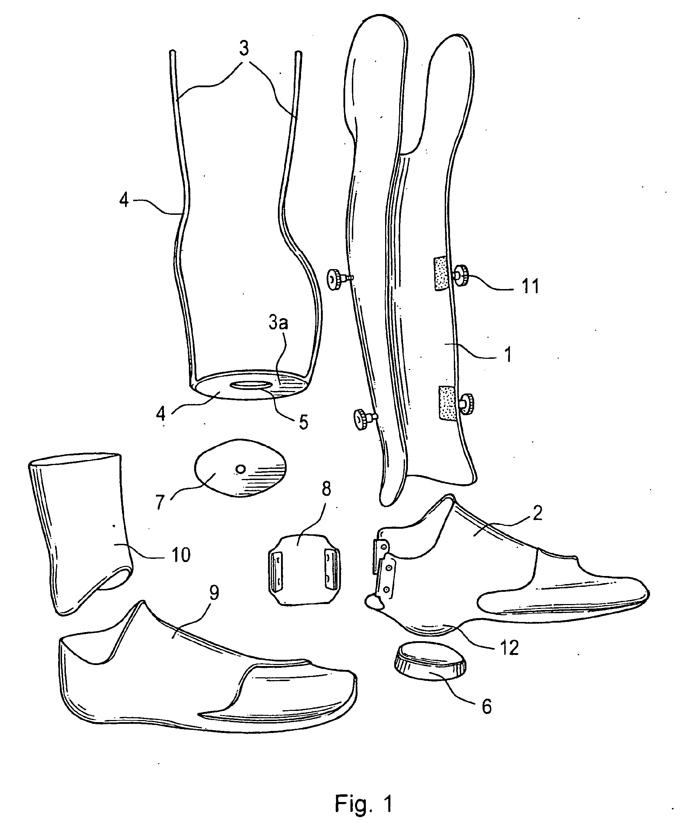

Figur 1 zeigt nebeneinander Teile einer auseinander genommenen ersten erfindungsgemäßen Orthese. Rechts oben ist eine Unterschenkelschale 1 aus Gießharzlaminat zur Aufnahme eines Unterschenkels gezeigt. Die Unterschenkelschale 1 ist dazu ausgelegt, die Knöchel zu umklammem und so die Sprungbeinrolle in der Knöchelgabel zu fixieren. Die Knöchel können dabei durch eine tubusförmige Innenhülle 10 aus Polyethylen geschützt werden; ergänzend oder alternativ auch mit einem ringförmigen Polymergelkissen (nicht gezeigt).

Der Fuß wird mit einer Fußschale 2 gehalten, welche den Fuß im Bereich der Mittelfußknochen vollständig umschließt. Die Fußschale 2 ist dabei ebenfalls mit einer Innenhülle 9 aus Polyethylen ausgekleidet. Um den Einstieg in die Fußschale 2 zu erleichtern, ist das fersenseitige Ende der Fußschale 2 offen. Es wird mit einer zu verschraubenden Abdeckung 8 nach dem Einstieg verschlossen.

Schienen 3 sind über ein Übergangsstück 3a zum Auftrittsbügel 4 miteinander verbunden, so dass insgesamt eine etwa U-förmige Gestalt ausgebildet wird. Das Übergangsstück 3a weist eine Aussparung 5 auf, um eine verschiebliche sowie lösbare und fixierbare Lagerung der Fußschale 2 zu ermöglichen. Die Unterschenkelschale 1 wird über die Verschraubung 11 zwischen den seitlichen Schienen 3 befestigt.

Eine Pfanne 6 mit einer flachen Seite und einer konvexen Seite wird mit ihrer flachen Seite auf das Übergangsstück 3a aufgelegt. Befestigt wird die Pfanne 6 auf dem Übergangsstück 3a mit einer Halteplatte 7 unterhalb des Auftrittsbügels, wobei die Halteplatte 7 und die Pfanne 6 miteinander verschraubt werden und die entsprechende Schraube (nicht gezeigt) durch die Aussparung 5 hindurchgreift. Bei gelockerter Schraube ist die Pfanne 6 frei in der Ebene des Übergangsstückes 3a, also in der Transversalebene, verschiebbar, soweit es die Aussparung 5 erlaubt.

Die Fußschale 2 weist eine konkave Auswölbung 12 auf, welche mit der Pfanne 6 korrespondiert. Wird die Fußschale 2 über die Auswölbung 12 in die Pfanne 6 gelegt, so kann die Fußschale 2 über die Auswölbung 12 entlang der Oberfläche der Pfanne 6 in allen Ebenen gedreht werden; die Auswölbung 12 fungiert sozusagen als Gelenkkopf eines Kugelgelenks. Befestigt wird die Fußschale 2 in der Pfanne 6 durch eine aus der Pfanne 6 senkrecht nach oben weisende Schraube (nicht gezeigt; diese Schraube kann identisch mit der durch die Aussparung 5 geführten Schraube sein).

Die Auswölbung 12 weist sohlenseitig ein Loch (nicht gezeigt) mit einem Durchmesser von wenigen Zentimetern auf. Auf das Loch, im Inneren der Fußschale 2, wird eine verschiebbare Aufnahme (nicht gezeigt) für die Schraube aus der Pfanne 6 aufgelegt. Die Aufnahme kann von der Form her einem Ausschnitt aus einer Kugeloberfläche entsprechen. Wenn die Schraube in die Aufnahme hinein geschraubt und ausreichend angezogen ist, wird die Fußschale 2 in der Pfanne 6 fixiert. Wird die Schraube gelockert, so kann die Fußschale 2 über die Auswölbung 12 in der Pfanne 6 in alle Raumrichtungen gedreht werden.

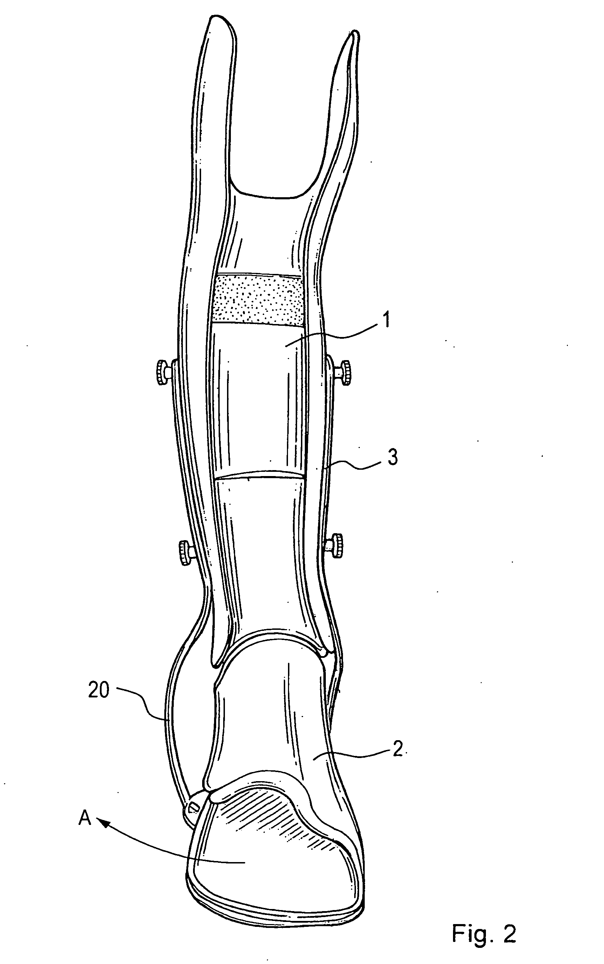

Figur 2 zeigt die erste erfindungsgemäße Orthese aus den in Figur 1 gezeigten Teilen; zusammengebaut wie dort beschrieben. Es handelt sich um eine Orthese zur Behandlung einer Deviation im Rückfuß mit einer Klumpfußkomponente.

Die Fußschale 2 und die Unterschenkelschale 1 sind in der Neutral-Null-Stellung dargestellt.

Nach dem Anziehen der Orthese wird die Fußschale 2 entlang der Pfeilrichtung A in der Transversalebene nach außen gedreht, um den pathologisch verkleinerten Winkel zwischen dem Fersenbein und dem Sprungbein zu korrigieren. Um eine hinreichende Redression des Fußes erreichen zu können, ist die außen liegende Schiene 3 ausgewölbt 20, um der Drehung A mehr Raum geben zu können.

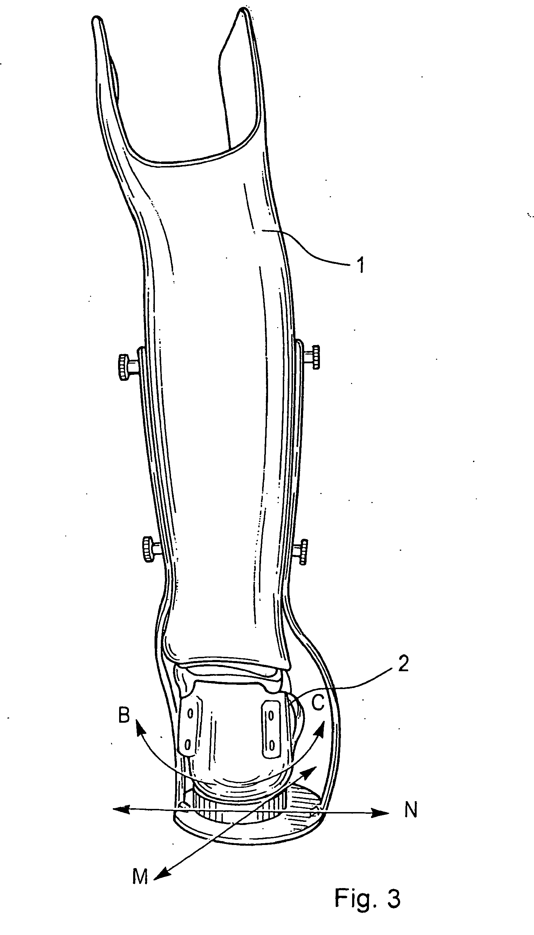

In Figur 3 ist die Orthese aus Figur 2 von hinten gezeigt. Pfeile M, N deuten an, dass die Pfanne 6 auf dem Auftrittsbügel 4 in der Transversalebene verschiebbar befestigt ist. Pfeile B und C deuten Drehungen zur Herstellung einer Pronation bzw. Supination an; die Fußschale 2 ist in der Frontalebene, also um ihre Länge um drehbar.

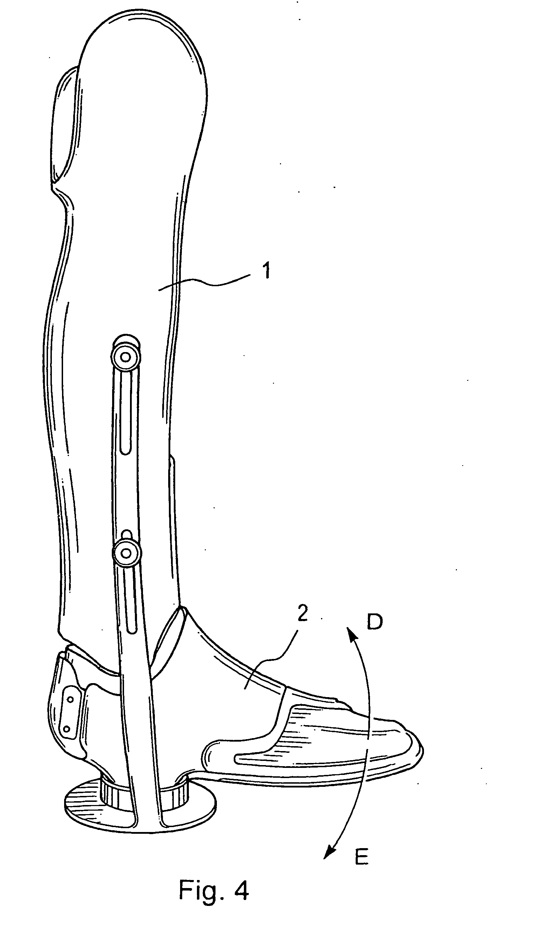

Figur 4 zeigt dieselbe Orthese von ihrer rechten Seite. Pfeile D und E deuten die Drehbarkeit der Fußschale 2 nach oben und nach unten in der Sagittalebene an.

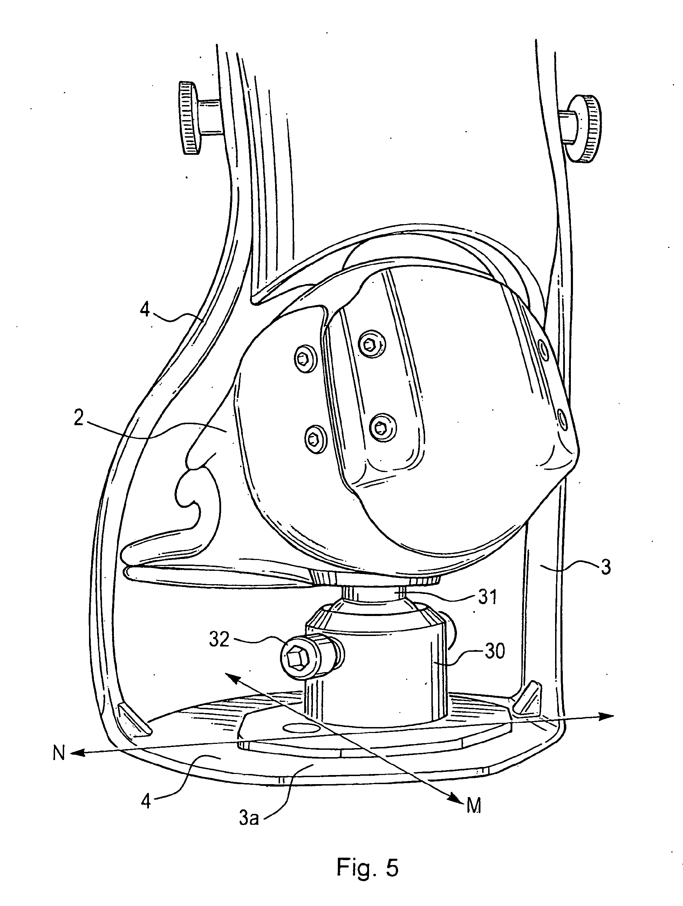

In Figur 5 ist der untere Abschnitt einer weiteren erfindungsgemäßen Orthese gezeigt. Die Fußschale 2 ist hier über ein anderes Gelenk 30 an dem Auftrittsbügel 4 abgestüzt. Auch hier ist das Gelenk 30 entlang des Auftrittsbügels 4 verschiebbar; vergleiche die Pfeile M, N. Bei dem Gelenk 30 handelt es sich um ein Kugelgelenk 30. Von dem Kugelgelenk 30 geht eine in jede Richtung kippbare Tragestange 31 ab, welche auch um ihre Längsachse drehbar ist. Fußschale 2 und Tragestange 31 sind starr miteinander verbunden. Durch die Drehbarkeit um die Längsachse der Tragestange 31 kann die Fußschale 2 in der Transversalebene gedreht werden. Durch Kippen der Tragestange 31 und Verschieben des Kugelgelenks 32 in der Transversalebene kann die Fußschale 2 auch in den anderen Ebenen gedreht werden.

An der Seite des Kugelgelenks 30 sind Schrauben 32 zu erkennen. Werden diese angezogen, so wird das Kugelgelenk 30 festgesetzt.

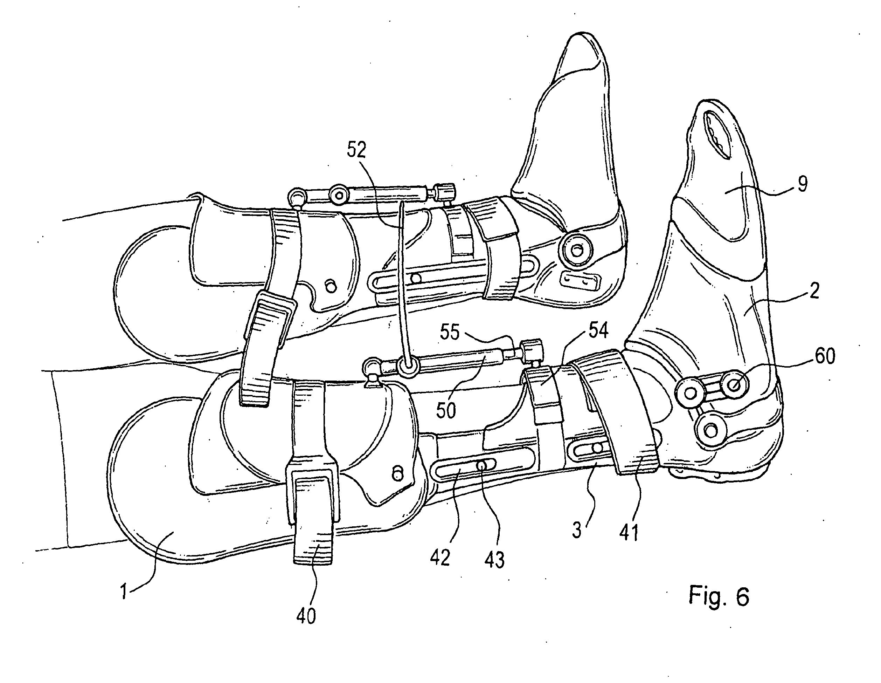

Figur 6 zeigt im Vordergrund eine an einen Unterschenkel angelegte Orthese zum Auseinanderziehen knöcherner Strukturen. Eine anatomisch formschlüssige mehrteilige Unterschenkelschale 1 umgibt den Unterschenkel kurz unterhalb des Knies. Die äußere Hülle der Unterschenkelschale 1 besteht aus einem Gießharzlaminat; sie ist ausgefüttert mit einer Innenhülle aus Polyethylen. Die Unterschenkelschale 1 ist an dem Schienbeinplateau und den Schienbeinkondylen abgestützt, um einen sicheren Halt zu gewähren. Mit einem die Unterschenkelschale 1 umgebenden Riemen 40 kann diese zur Verbesserung des Halts an den Unterschenkel gedrückt werden.

Der Fuß wird von einer anatomisch formschlüssigen Fußschale 2, bestehend aus dem gleichen Gießharzlaminat wie die Unterschenkelschale 1, gehalten, welche den Fuß im Bereich der Mittelfußknochen ringförmig umfasst. Eine Innenhülle 9 aus Polyethylen zur Einbettung des Fußes ist hier gut zu erkennen.

Im Bereich der Ferse bzw. Knöchel erkennt man eine außen liegende Fußschalenaufhängung 60, welche ein Verkippen der Fußschale 2 relativ zur Transversalebene erlaubt.

Seitliche Schienen 3 führen ausgehend von der Fußschale 2 in Richtung Knie (man erkennt nur die rechte Schiene 3 im Vordergrund; die linke Schiene ist verdeckt durch den Unterschenkel). Die Schienen 3 sind über die Fußschalenaufhängung 60 derart an der Fußschale 2 befestigt, dass sie in Streckrichtung unbeweglich sind.

Die seitlichen Schienen 3 enden etwa mittig zwischen dem Fuß und dem Knie. In diesem Bereich der Schienen 3 befindet sich auf jeder Seite ein etwa 5 cm langer Führungsspalt 42. Die mehrteilige Unterschenkelschale 1 greift auf jeder Seite über einen Führungsstift 43 in den entsprechenden Führungsspalt 42 ein. Werden Fußschale 2 und Unterschenkelschale 1 in Streckrichtung gegeneinander verschoben, so wird die Bewegung durch die Schienen 3 und die in die entsprechenden Führungsspalte 42 eingreifenden Führungsstifte 43 geführt.

Die beiden Schienen 3 sind etwa mittig durch einen um das Schienbein herumgreifenden metallischen Bügel 54 miteinander verbunden. Ein pneumatischer Arbeitszylinder 50 ist im wesentlichen parallel zu der Streckrichtung ausgerichtet und einerseits an dem Bügel 54 befestigt und andererseits weiter oben an der Unterschenkelschale 1. Durch Umlegen eines Hebels 52 an dem pneumatischen Arbeitszylinder 50 kann die Kolbenstange 55 ausgefahren werden. Der Hebel 52 ist in zwei Einstellungen arretierbar. Bei einer der Einstellungen ist die Kolbenstange 55 maximal ausgefahren; bei der anderen Einstellung ist die Kolbenstange 55 weitgehend eingezogen. Entsprechend werden bei maximal ausgefahrener Kolbenstange 55 die Fußschale 2, vermittelt über den Bügel 54 und die Schienen 3, und die Unterschenkelschale 1 auseinander gedrückt. Bei der anderen Einstellung des Hebels 52 werden die Fußschale 2 und die Unterschenkelschale 1 wieder zusammengezogen.

Anstatt mit einem Arbeitszylinder 50 kann der Abstand zwischen der Fußschale 2 und der Unterschenkelschale 1 auch über eine Gewindestange, Zahnstange oder über ein Schneckengetriebe (jeweils nicht gezeigt) eingestellt werden. Mit entsprechenden Ausführungsformen kann der Abstand zwischen der Fußschale 2 und der Unterschenkelschale 1 auch frei wählbar eingestellt werden, soweit ein minimaler Abstand nicht unter- bzw. ein maximaler Abstand nicht überschritten wird.

Der Abstand zwischen der Fußschale 2 und der Unterschenkelschale 1 wird über den Arbeitszylinder 50 federnd-nachgiebig eingestellt. Wird die Orthese bspw. in Streckrichtung zusammengerückt, so wird sie aufgrund der federnd-nachgiebigen Abstandseinstellung etwas gestaucht. Lässt die Belastung nach, wird wieder der eingestellte Abstand angenommen.

Über den Arbeitszylinder 50, etwa einen hydropneumatischen Zylinder, kann eine Kraft vorgegeben werden, mit der die Befestigungseinrichtungen auseinander gedrückt werden können, beispielsweise 200 bis 600 Newton. Kleinere und größere Werte sind grundsätzlich auch denkbar.

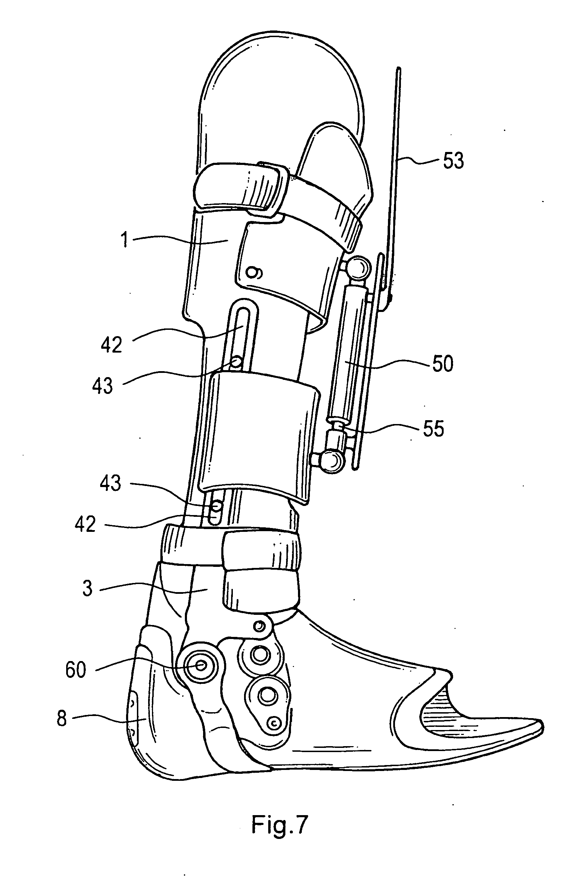

Figur 7 zeigt eine weitere Orthese zum Auseinanderziehen knöcherner Strukturen. Auch diese weist, der Orthese aus Figur 6 vergleichbar, eine entsprechende Unterschenkelschale 1, Fußschale 2 und an der Fußschale 2 befestigte Schienen 3 auf.

Im Unterschied zu der Orthese aus Figur 6 weisen die Schienen 3 hier zwei Führungsspalte 42 auf, in die die Unterschenkelschale 1 über jeweils einen Führungsstift 43 eingreift.

Auch hier wird ein um das Schienbein vorn herumgreifender Bügel 54 verwendet. Hier ist er allerdings aus einem Gießharzlaminat hergestellt, außerdem verdeckt er die Schienen 3 jeweils zwischen den beiden Führungsspalten 42.

Im hinteren Teil der Fußschale 2 erkennt man eine Fersenklappe 8, die für einen leichten Ein- und Ausstieg entfernt werden kann.

Der Abstand zwischen der Unterschenkelschale 1 und der Fußschale 2 wird auch hier über einen pneumatischen Arbeitszylinder 50 eingestellt. Über einen Hebel 53 wird auch hier eine Kolbenstange 55 aus dem Arbeitszylinder heraus gedrückt oder in diesen eingezogen. Der Hebel 53 ist exzentrisch an einem Arretierblech 51 gelagert und wird zur Seite hin betätigt. Sowohl bei ausgefahrener als auch bei eingezogener Kolbenstange wird der Hebel 53 mittels Aussparungen (nicht erkennbar) an dem Arretierblech 51 fixiert.

Bei fixiertem Hebel 53 ist der Abstand zwischen der Fußschale 2 und der Unterschenkelschale 1 unveränderlich eingestellt.

Figur 8 zeigt eine Orthese zur Behandlung pathologischer Deviationen in Rückfuß und zum Auseinanderziehen knöcherner Strukturen. Der obere Teil dieser Orthese mit Unterschenkelschale 1, Riemen 40, Führungsspalten 42 in Schienen 3, Führungsstift 43, Arbeitszylinder 50, Hebel 52, Bügel 54 und Kolbenstange 55 entspricht im wesentlichen dem Oberteil der Orthese aus Figur 7. Der untere Teil des Arbeitszylinders 50 ist, wie in Figur 7, über Kolbenstange 55 und den Bügel 54 an den Schienen 3 befestigt. Der obere Teil des Arbeitszylinders 50 ist an der Unterschenkelschale 1 befestigt (die Verbindungen zwischen dem Bügel 54 und dem Arbeitszylinder 50 beziehungsweise zwischen der Unterschenkelschale aus 1 und dem Arbeitszylinder 50 sind nicht explizit eingezeichnet). Weiter ist eine Fußschale 2 wie in Figur 7 vorhanden.

Der untere Abschnitt der in Figur 8 gezeigten Orthese entspricht der in den Figuren 1 und 4 gezeigten Ausführungsform. Auch hier bilden die Schienen 3 einen Auftrittsbügel 4 aus. Der Auftrittsbügel 4 ist unten zu einer Halteplatte 7 verbreitert, auf der, wie oben erläutert, eine Gelenkpfanne 6 aufgeschraubt ist. Auch hier ist die Fußschale 2 formschlüssig in der Pfanne 6 beweglich befestigt.

Die Schienen 3 weisen am unteren Ende der Unterschenkelschale 1 beziehungsweise oberhalb der Fußschale 2 ein Gelenk 70 auf, so dass der untere Teil der Schienen 3 relativ zu deren oberen Teil nach vorne und hinten und andersherum verkippt werden kann. Die Drehachse korrespondiert mit dem oberen Sprunggelenk. Der Freiraum zwischen Fußschale 2 und Unterschenkelschale 1 erlaubt eine entsprechende Bewegung. Das Gelenk 70 ist lösbar und fixierbar für jeden einstellbaren Kippwinkel. Das lös- und fixierbare Gelenk 70 kann auch einen Motor zum Kippen und Fixieren umfassen. Alternativ zu einem einen Motor aufweisenden Gelenk 70 können die Fußschale 2 und die Unterschenkelschale 1 über einen Antrieb miteinander verbunden sein, welcher über eine Stange die Fußschale 2 und die Unterschenkelschale 1 anpackt. Die Stange ist dabei beispielsweise im Mittelfußbereich der Fußschale 2 und im unteren Bereich der Unterschenkelschale 1 befestigt (nicht gezeigt).

Mit dem Gelenk 70 wird die Möglichkeit zur Stellungskorrektur erweitert. Außerdem kann über das Gelenk 70 und den ggf. zusätzlich vorhandenen Motor das Sprunggelenk mobilisiert werden. So kann beispielsweise ein Einsteifen nach einer Operation, etwa einer Klumpfußoperation, verhindert werden. Weiter kann über das Gelenk 70 und einen entsprechenden Motor auch Arthrose therapiert werden. Hier ist es besonders vorteilhaft, dass Fußschale 2 und Unterschenkelschale 1 dabei auch auseinander gezogen werden können. Dies vereinfacht die Bewegungstherapie und vermeidet auch Schmerzen.

Insgesamt ermöglicht diese Orthese sämtliche oben angeführten Bewegungen und Drehungen der Fußschale 2 relativ zu der Unterschenkelschale 1. Außerdem können bei dieser Orthese Unterschenkelschale 1 und Fußschale 2 auseinander gezogen werden. Zusätzlich kann die Fußschale 2 über das Gelenk 70 nach vorne und nach hinten verkippt werden.

### Bezugszeichenliste

- 1: Unterschenkelschale
- 2: Fußschale
- 3: seitliche Schiene
- 3a: Übergangsstück
- 4: Auftrittsbügel
- 5: Aussparung
- 6: Pfanne
- 7: Halteplatte
- 8: Fersenklappe
- 9: Innenhülle
- 10: Innenhülle
- 11: Verschraubung
- 12: Auswölbung
- 20: Auswölbung
- 30: Gelenk
- 31: Tragestange
- 32: Schraube40 Riemen
- 41: Riemen
- 42: Führungsspalt
- 43: Führungsstift
- 50: Arbeitszylinder
- 51: Arretierblech
- 52: Hebel
- 53: Hebel
- 54: Bügel
- 55: Kolbenstange
- 60: Fußschalenaufhängung
- 70: Gelenk

- A - E: Drehrichtung

- M, N: Verschieberichtung

## Patentansprüche

1. Orthese zur Behandlung pathologischer Deviationen im Rückfuß mit
einer oberen Fixiereinrichtung (1) und
einem Fußteil (2), das zur Aufnahme der subtalaren Fußplatte eingerichtet ist und relativ zu der oberen Fixiereinrichtung (1) in der Transversalebene zwischen mindestens zwei Ausrichtungen drehbar und in jeder der Ausrichtungen lösbar fixierbar ist,
**dadurch gekennzeichnet, dass** die obere Fixiereinrichtung (1) zum Fixieren der Sprungbeinrolle in der Knöchelgabel ausgelegt ist.

2. Orthese nach Anspruch 1, bei der das Fußteil (2) in der Transversalebene zweidimensional verschiebbar und in jeder Position lösbar fixierbar ist.

3. Orthese nach einem der vorangehenden Ansprüche, bei der das Fußteil (2) auch in der Sagittal- und/oder Frontalebene drehbar und lösbar fixierbar ist.

4. Orthese nach einem der vorangehenden Ansprüche, bei der die obere Fixiereinrichtung (1) ein Schalenteil (1) für den Unterschenkel aufweist, welches den Innen- und den Außenknöchel umklammert.

5. Orthese nach einem der vorangehenden Ansprüche, bei der das Fußteil (2) ein Schalenteil (2) zum Halten der subtalaren Fußplatte aufweist.

6. Orthese nach einem der vorangehenden Ansprüche, bei der das Fußteil (2) an einer Halterung (3,4) befestigt ist, die ihrerseits an der oberen Fixiereinrichtung (1) befestigt ist, und die Halterung (3,4) als U-förmiger Auftrittsbügel (4) ausgebildet ist, dessen beide Schenkel (3) an der oberen Fixiereinrichtung (1) befestigt sind und dessen geschlossenes Ende sich unter dem Fußteil (2) befindet, wobei das Fußteil (2) an dem Auftrittsbügel mittels eines Gelenks (6, 12, 30) drehbar gestützt ist.

7. Orthese nach Anspruch 6, bei der das Gelenk (6, 12, 30) ein Kugelgelenk ist.

8. Orthese nach Anspruch 6 oder 7, bei der das Gelenk (6, 12, 30) in der Transversalebene verschiebbar an dem Auftrittsbügel (4) befestigt ist.

9. Orthese nach einem der vorangehenden Ansprüche zum gestreckten Halten, insb. Auseinanderziehen, flexibler knöcherner Strukturen eines Körpers, insbesondere eines intraartikulären Spalts,
mit einer ersten Befestigungseinrichtung (1, 2) zum Fixieren der Orthese an dem Körper auf einer Seite der knöchernen Struktur,
einer zweiten Befestigungseinrichtung (2, 1) zum Fixieren der Orthese an dem Körper auf der, bezogen auf die Streckrichtung, anderen Seite der knöchernen Struktur, und
einer die erste Befestigungseinrichtung (1, 2) und die zweite Befestigungseinrichtung (2, 1) miteinander verbindenden Einstelleinrichtung (50), welche dazu ausgelegt ist, durch ihre Betätigung wahlweise einen verkürzten oder einen verlängerten Abstand zwischen der ersten und der zweiten Befestigungseinrichtung (1, 2; 2, 1) herzustellen.

## Claims

1. An orthosis for handling pathological deviations in the hind-foot, comprising an upper fixing means (1) and
a foot part (2) which is equipped for receiving the subtalar foot plate and is rotatable relative to the upper fixing means (1) in the transversal plane between at least two orientations and can releasably be fixed in each of the orientations,
**characterized in that** the upper fixing means (1) is designed for fixing the trochlea of the talus in the mortice.

2. The orthosis according to claim 1, in which the foot part (2) can be displaced in two dimensions in the transversal plane and can releasably be fixed in each position.

3. The orthosis according to any of the preceding claims, in which the foot part (2) can be rotated and releasably be fixed also in the sagittal and/or frontal plane.

4. The orthosis according to any of the preceding claims, in which the upper fixing means (1) includes a shell part (1) for the lower leg, which clasps the inner and the outer malleolus.

5. The orthosis according to any of the preceding claims, in which the foot part (2) includes a shell part (2) for holding the subtalar foot plate.

6. The orthosis according to any of the preceding claims, in which the foot part (2) is attached to a holder (3, 4) which in turn is attached to the upper fixing means (1), and the holder (3, 4) is formed as U-shaped step-on stirrup (4) whose two legs (3) are attached to the upper fixing means (1) and whose closed end is located below the foot part (2), wherein the foot part (2) is rotatably supported on the step-on stirrup by means of a joint (6, 12, 30).

7. The orthosis according to claim 6, in which the joint (6, 12, 30) is a ball joint.

8. The orthosis according to claim 6 or 7, in which the joint (6, 12, 30) is attached to the step-on stirrup (4) so as to be movable in the transversal plane.

9. The orthosis according to any of the preceding claims for holding stretched, in particular for pulling apart flexible bony structures of a body, in particular of an intra-articular gap, comprising a first fixing means (1, 2) for fixing the orthosis at the body on one side of the bony structure,
a second fixing means (2, 1) for fixing the orthosis at the body on the other side of the bony structure, relative to the stretching direction, and
an adjusting means (50) connecting the first fixing means (1, 2) and the second fixing means (2, 1) with each other, which is designed to selectively provide a reduced or increased distance between the first and the second fixing means (1, 2; 2, 1) by actuating the same.

## Revendications

1. Orthèse pour le traitement de déviation pathologique dans l'arrière du pied, comprenant un moyen de fixation supérieur (1) et
une partie de pied (2), qui est conçue pour recevoir la plaque de pied sous l'astragale et est susceptible d'être fixée de manière rotative entre au moins deux orientations par rapport au moyen de fixation supérieur (1) dans le plan transversal et de manière détachable dans chacune des orientations,
**caractérisée en ce que** le moyen de fixation supérieur (1) est conçu pour fixer la tête de l'astragale dans la fourche malléolaire.

2. Orthèse selon la revendication 1, dans laquelle la partie de pied (2) est susceptible d'être fixée avec possibilité de translation dans les deux dimensions dans le plan transversal, et d'être détachée dans chaque position.

3. Orthèse selon l'une des revendications précédentes, dans laquelle la partie de pied (2) est également fixée avec possibilité de rotation dans le plan sagittal et/ou frontal, et de manière détachable.

4. Orthèse selon l'une des revendications précédentes, dans laquelle le moyen de fixation supérieur (1) comprend une partie en coque (1) pour la jambe, qui enserre la malléole intérieure et la malléole extérieure.

5. Orthèse selon l'une des revendications précédentes, dans laquelle la partie de pied (2) comprend une partie en coque (2) pour retenir la plaque de pied sous l'astragale.

6. Orthèse selon l'une des revendications précédentes, dans laquelle la partie de pied (2) est fixée sur une monture (3, 4) qui est fixée à son tour sur le moyen de fixation supérieur (1), et la monture (3, 4) est réalisée sous forme d'un arceau de marche en forme de U (4), dont les deux branches (3) sont fixées au moyen de fixation supérieur (1), et dont l'extrémité fermée se trouve au-dessous de la partie de pied (2), de sorte que la partie de pied (2) est soutenu sur l'arceau de marche avec possibilité de rotation au moyen d'une articulation (6, 12, 30).

7. Orthèse selon la revendication 6, dans laquelle l'articulation (6, 12, 30) est une articulation à rotule.

8. Orthèse selon la revendication 6 ou 7, dans laquelle l'articulation (6, 12, 30) est fixée sur l'arceau de marche (4) avec possibilité de translation dans le plan transversal.

9. Orthèse selon l'une des revendications précédentes, destinée à maintenir en situation étirée, en particulier en écartement les unes des autres, des structures osseuses flexibles d'un corps, en particulier d'un intervalle interarticulaire,
comprenant un premier moyen de fixation (1, 2) pour fixer l'orthèse sur le corps sur un côté de la structure osseuse,
un second moyen de fixation (2, 1) pour fixer l'orthèse sur le corps de l'autre côté, par référence à la direction d'allongement, de la structure osseuse, et
un moyen de réglage (50), qui relie l'un à l'autre le premier moyen de fixation (1, 2) et le second moyen de fixation (2, 1) et qui est conçu pour établir du fait de son actionnement au choix un écartement raccourci ou un écartement allongé entre le premier et le second moyen de fixation (1, 2 ; 2, 1).
